# EUROPEAN PATENT SPECIFICATION

(11) **EP 2 926 736 B1**
(45) Date of publication and mention of the grant of the patent: **17.06.2020**
(21) Application number: 14162769.5
(22) Date of filing: 31.03.2014
(51) Int. Cl.: A61B 8/00, A61B 8/08

(54) **Apparatus and method for ultrasound image acquisition, generation and display**
Vorrichtung und Verfahren zur Ultraschallbilderfassung, -erzeugung und -anzeige
Appareil et procédé d'acquisition d'images ultraacoustiques, de génération et d'affichage

(43) Date of publication of application: 07.10.2015
(73) Proprietor: Esaote S.p.A., 16152 Genova (IT); MedCom GmbH, 64283 Darmstadt (DE)
(72) Inventor: Forzoni, Leonardo, I-51100 Pistoia (IT); De Beni, Stefano, I-16123 GENOVA (IT); Kolev, Velizar, 64285 Darmstadt (DE); Sakas, Georgios, 64285 Darmstadt (DE)
(74) Representative: Karaghiosoff, Giorgio Alessandro

(56) References cited:
- WO-A1-2013/035393
- US-A1- 2007 167 709
- US-B1- 6 500 119

## Description

The present invention relates to a method of ultrasound image acquisition, generation and display.

Ultrasound imaging has become an important and popular diagnostic approach, due to its wide range of applications.

Especially in medicine, this kind of diagnostic approach is widely used, due to its non-invasive and non-destructive nature.

Modern ultrasound apparatus are commonly used to generate 2D or 3D ultrasound images, reproducing the internal characteristics of an anatomic region of a body being examined, such as an organ.

The ultrasound system transmits ultrasound signals to the body under examination and receives echo signals, thereby forming a 2D (two-dimensional) or 3D (three-dimensional) image, which 2D or 3D image is displayed on a display.

In the prior art, the position of the probe at which image acquisition occurs is immediately recognized due to the simultaneity between image acquisition and display.

When the image is displayed after acquisition and also, as is increasingly often the case, when image display and acquisition are carried out by different operators, the physician that analyzes the image can only recognize the probe position at which image acquisition occurred from the displayed image, based on his/her expertise.

Nevertheless, in certain cases, it may be difficult to recognize the position of the probe from the displayed image, and this may lead to uncertainty about the location of any surgery or any further diagnostic examination.

This particularly occurs in breast imaging, where tissues are soft and may undergo deformation and displacement.

The various diagnostic techniques for breast imaging, such as mammography, breast tomosynthesis, breast MRI, ductography, MBI (Molecular Breast Imaging), breast PET, require special patient positioning or compression of the breast.

Therefore, an image obtained using an imaging mode can be hardly related to an image obtained using a different imaging mode.

Studies have been conducted on breast deformation based on patient positioning or controlled mechanical stresses, in view of creating a reliable breast representation model, but these studies have not led to satisfactory results, due to the high heterogeneity of breast characteristics which not only change from patient to patient, but also from the right breast to the left breast of the same patient.

In breast surgery, when the first diagnostic examination detects an abnormality, biopsy is usually performed, typically with a stereotactic needle biopsy procedure.

Since stereotactic references might be different in later surgery, if any, a small amount of carbon is deposited along the hole formed by the biopsy needle, for easy X-ray recognition by the surgeon.

Nevertheless, this procedure requires the patient to be exposed to potentially hazardous ionizing radiation.

Therefore, there is a need, yet unfulfilled by prior art imaging methods and apparatus, of accompanying a displayed ultrasound image with information about probe positioning at the time of acquisition.

Documents US6500119B1 and US2007/0167709A1 disclose the acquisition and visualisation of ultrasound images along with an indication of the probe position.

The method of the present invention is aimed at obviating the drawbacks of the prior art by means of simple and inexpensive arrangements, which can avoid risks for the patient while ensuring clear interpretation of the acquired images.

The present invention fulfils the above objects by a method according to claim 1.

This will provide a preferably but not necessarily stylized map, or bodymark, representing the outer surface of the anatomic region of interest.

The map is displayed at the same time as the ultrasound image, whereby the position of the probe is related to the marked point.

Therefore, the physician receives a qualitative, clear indication of the point of the body being examined, from which the image was acquired by the ultrasound probe.

This may improve the diagnostic confidence level and reduce imaging times, thereby reducing costs and increasing the usability of the acquisition system.

The method may be used in any anatomic region, advantageously the breast, the musculoskeletal apparatus and the lower limbs.

The breast is an organ in which very few reference points may be recognized in a scan.

For this reason, and for the fact that a complete scan of breast would be required to ensure a proper accuracy level, a clear marking of the image acquisition point is highly useful for an operator and for those that will later view and analyze the image.

In the case of the musculoskeletal apparatus, acquisition point marking is useful for examination and follow-up, especially after pharmacological and/or surgical treatments of rheumatic diseases, for associating the image with the correct probe position on the anatomic region of interest.

As for the use with lower limbs, the method is useful for venous mapping and surgery planning, or the like.

This allows the physician to create a detailed map of the vein to be treated, which is marked on the bodymark that represents the limb being examined.

In one exemplary embodiment, the step of generating the map comprises detecting the spatial relationship among points of interest on the outer surface of the above mentioned anatomic region and reproducing such spatial relationship among points on the map corresponding to those points of interest.

Thus, a standard map may be generated, which will be later scaled to faithfully conform to the surface conformation of the anatomic region of interest.

Otherwise, the generated map may include the spatial relationships among points corresponding to the spatial relationships among points of the surface of the anatomic region of interest as detected.

In a first variant embodiment, the map is a two-dimensional map.

Thus, the map may represent a plane or the projection of an anatomic surface on a reference plane.

In a second variant embodiment, the map is a three-dimensional map.

Thus, the map may be displayed in different modes and from different points of view, e.g. by rendering, to provide more accurate indication to the operator.

The map may have a detail level from the most realistic to the most stylized.

In more stylized forms, the map will be conveniently conformed to add an indication about whether the anatomic district is located in the left or right side of the patient.

One or more additional images are displayed at the same time as the ultrasound image and the map, the detected position of the above mentioned probe being marked on said one or more additional images.

These additional images may be also ultrasound images or images acquired under different imaging modes.

In a particularly advantageous embodiment, the additional images are two images acquired along two planes more or less perpendicular to the plane represented in the two-dimensional map, and more or less perpendicular to each other (such as in the case of mammography).

Thus, the two-dimensional map identifies a first plane with the probe position marked thereon, whereas the additional images identify two planes perpendicular to the plane of the map and perpendicular to each other, also having the probe position marked thereon.

Therefore, the operator may view the acquired ultrasound image as well as the position of the ultrasonic probe at the time of acquisition, as marked on three planes more or less perpendicular to each other, thereby receiving an accurate indication of such position.

In one embodiment, in which a three-dimensional map is provided, the map is generated by acquiring at least one three-dimensional ultrasound volume of the anatomic region, and only retaining the surface information, i.e. preferably the information about the skin.

Therefore, the three-dimensional map will be generated after discarding any tissue information of the three-dimensional ultrasound volume except the skin information, with the probe position as detected at the time of acquisition of the displayed ultrasound image being marked on such three-dimensional map.

In one improvement of this embodiment, two or more three-dimensional ultrasound volumes are acquired and joined together by a merging process, to provide said three-dimensional ultrasound volume.

This will afford mapping of larger areas, by separately scanning preferably adjacent or overlapping portions to be merged into a single volume.

The invention also relates to an apparatus for ultrasound image acquisition, generation and display according to claim 8.

In a preferred embodiment, the position of the ultrasound probe during image data acquisition is detected by a tracking system.

The tracking system may be of any currently known type, and is preferably of electromagnetic type.

These and other features and advantages of the present invention will appear more clearly from the following description of a few embodiments, illustrated in the annexed drawings, in which:
Fig. 1 shows the ultrasound image and the map on display with the mammography reference image on the side and the bodymark related to the coronal view of the left breast;
Fig. 2 shows the position of the probe at the time of acquisition of the ultrasound image;
Figs. 3 and 4 show a transformation between the representation of the organ in examination and the real organ, which is subject to shape changes or bend;
Fig. 5 shows a graphical representation of a possible deformation algorithm;
Fig. 6 and 7 show two exemplary embodiments of the method of reconstructing a three-dimensional map;
Figure 8 shows a graphical scanning protocol.

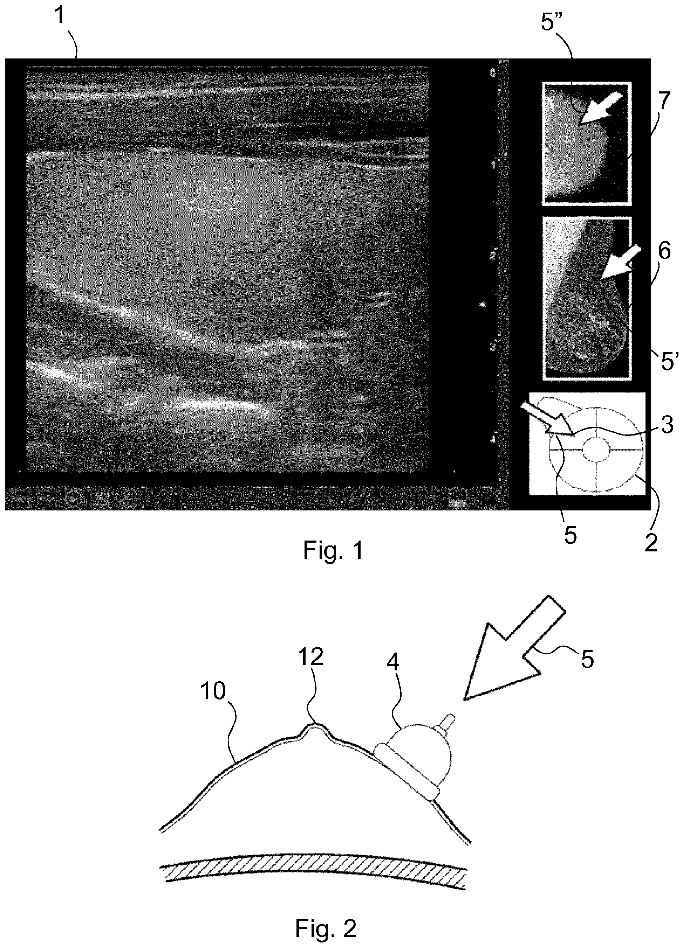

Figure 1 shows the final output of the method of the present invention, which comprises acquiring an ultrasound image 1 by an ultrasound probe 4, generating a map 2 of the outer surface of the anatomic region being examined, detecting the position of the probe 4 during acquisition of the image 1, locating the point 3 on the map, corresponding to the detected position of the probe 4, and displaying the map 2 with the point 3 marked thereon, at the same time as the ultrasound image 1 is displayed.

The position of the ultrasound probe during ultrasound image acquisition is detected by a tracking system, preferably of electromagnetic type.

Advantageously, the tracking system has means for correcting the measured position, which operate by always relating the position of the probe to that of the patient.

Thus, the patient is the reference of the tracking system, and the calibration/registration is maintained even when he/she moves/is moved.

This occurs considering the movements of the anatomic region as rigid and with no bending, distortion or deformation.

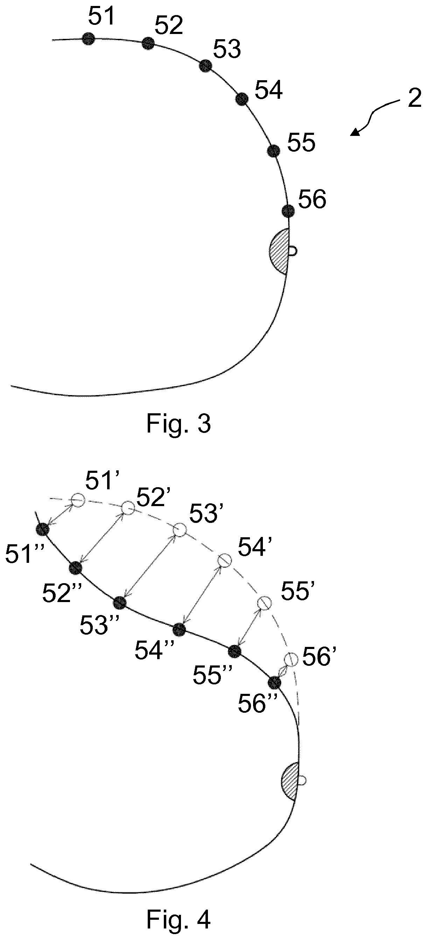

As an alternative, the shape change or bending of the examiner organ is considered, as shown in figures 3 to 5.

Figure 2 shows the corresponding position of the probe 4, when it is positioned on the anatomic region of interest, particularly a breast 10.

In the method of the present invention, an operator initially marks a series of points on the surface of the anatomic region of interest, i.e. the breast 10, by a marking device that communicates with the tracking system, preferably according to a preset time sequence.

The marking device may consist of the ultrasound probe or a separate device.

Thus, the spatial relationship among points of interest on the outer surface of the anatomic region is detected.

Then, the spatial relationship among the points on the map 2 corresponding to the points of interest is reproduced.

In the case of the breast, the operator marks the position corresponding to the nipple 12 and to four points on the breast perimeter, i.e. an inner point (i.e. facing toward the other breast), a low point, a lateral point and an underarm point, relative to the nipple 12.

In marking is made in the right time sequence, the system can determine whether the marking sequence concerns the right breast or the left breast.

Then, the system displays the correct map of the breast of the proper side of the body being examined.

Nevertheless, the operator is always allowed to change the side of the body by loading a map of the opposite side, or by deleting the procedure if the marking steps, i.e. the point calibration/selection steps have not had a successful or desired result.

Therefore, the acquired points have a direct relationship to points that are already present on the map, to ensure point-to-point correlation and to indicate the scaling factor of the map, both in relative terms among the various points, and in general terms, i.e. relative to the actual breast size.

The map 2 as shown in the figure represents a coronal plane projection and also comprises the underarm region.

The presence of the underarm region allows immediate recognition of the body side of the breast being examined.

The indication of ultrasound image acquisition positions also in the axillary cavity is very important. Then, the map 2 is re-calibrated according to the relative distances detected among the marking points on the real breast of the patient.

The shape and sizes of the map are substantially unchanged, but the relative sizes are changed and recalibrated.

After this registration step and approval thereof by the operator, the operator may start a scan to obtain the ultrasound image, with the position of the probe on the breast being automatically marked on the map, and the relative position being recalibrated according to the real size of the breast being examined.

The mark may consist of an arrow-shaped icon 5, but other shapes may be envisaged, such as a colored dot.

In an advantageous embodiment, marking is indicated by an icon that represents the probe and advantageously corresponds to the type of probe in use, e.g. a linear, convex or another type of probe.

The position information is of qualitative type and is of help for the operator, i.e. for follow-up or, more likely, in the cases in which an operator performs the scan and the diagnosis is suggested by a physician.

The map may also be a valuable option for a surgeon to understand the relative position of a lesion or a target within the mass of the breast.

The operator still has the option of changing the position of the icon 5 on the map 2, both in real time and at a later time, on the stored image.

For this purpose, a manual icon 5 positioning feature is provided, which can be automatically actuated before saving the image 1, and which allows manual correction of the position of the icon 5 on the map 2, for marking the position of the probe 4 during the scan.

As an improvement, two additional images 6 and 7 are displayed at the same time as the ultrasound image 1 and the map 2, the detected position of the probe 4 being marked on each of the two additional images 6 and 7.

The additional images 6 and 7 are two images acquired along two planes perpendicular to the plane represented in the two-dimensional map, and perpendicular to each other, i.e. the sagittal plane and the axial or transverse plane.

The additional images 6 and 7 may either be ultrasound images or be acquired in any other imaging mode, such as MRI or TC.

Advantageously, the images 6 and 7 as shown in figure 1 are mammogram images.

During mammography, the breast is compressed to obtain two breast images, on the sagittal plane and the axial or transverse plane respectively. Thus, mammogram scans may be used as additional maps representing the two planes in addition to the coronal plane as shown in the map 2.

The calibration/registration procedure is carried out as described above concerning the map 2.

When the mammogram images are also loaded, the three views are shown on the screen, and once the calibration step is complete, the relative position of the prove is marked on each of the two additional images, using two icons 5' and 5" respectively.

For musculoskeletal applications, the map 2 is loaded using the actual dimensions or the limb or extremity or shoulder being examined. An appropriate map 2 must be created for this purpose.

A second imaging mode, such as MRI, with one or more images on different planes, may be provided to increase the detail level of the displayed probe position during the scan.

The images obtained from the additional imaging mode, preferably but not necessarily MRI, may be included in a single three-dimensional image derived from a single scan, for extraction and display of two images on the sagittal and transverse planes respectively.

Instead of or in addition to the above, the three-dimensional image may be entirely displayed on screen.

In lower limb applications for venous mapping and follow up, the venous system is the main target of the examination, whereby ultrasound imaging is the most suitable diagnostic imaging mode, also used for surgery or treatment.

A possible imaging mode for displaying additional images may be, for example, angiography.

In musculoskeletal system and lower limb applications, the anatomic region under examination must be in the position as described in the map and/or the images acquired in the additional imaging mode.

For example, no hand or limb contraction is allowed during the ultrasound scan, otherwise quality localization of the probe on the map or the additional images would no longer be valid.

Likewise, correspondence is required between the map and the images acquired with the additional imaging mode. Therefore, if the map after calibration/registration represents a fully extended arm, then the images acquired through the second mode, e.g. MRI, should also show an arm in the same position.

This also applies to hands, elbows and partially also to the shoulder, due to the restricted mobility of the latter.

In these cases, considering the higher probability of errors and inaccuracies, it will be highly convenient for the operator to be able to check the consistency of the probe position relative to the position marked on the map and on the additional images, and to manually correct such indication.

Figure 3 and 4 show a transformation between the representation of the organ in examination and the real organ, which is subject to shape changes or bend.

Figure 3 represents the imagine of the considered breast 10 as represented for instance on the mammography exam, loaded on the Ultrasound system as a reference image. Figure 4 represents the real breast of the examined patient: being not stretched within the mammography, it's more relaxed loosing the almost semi-spherical shape as on figure 3.

The points 51 to 56 on figure 3 are the ones selected by the operator in order to adapt the bodymark or map 2, here represented by the mammography sagittal view, to the real organ scanned of the patient.

Regarding the real shape of the organ scanned, the operator indicates the points from 51" to 56" and he/she links those to the points from 51 to 56 on the bodymark or map 2, i.e. the reference image of figure 3.

By doing this, while moving the probe on the real profile of the real examined organ of figure 4, the representation of the probe on the bodymark or map 2 will move on the line intercepted by the points from 51 to 56, i.e. the same as moving in the real world on the hypothetical points from 51' to 56' as indicated on figure 4.

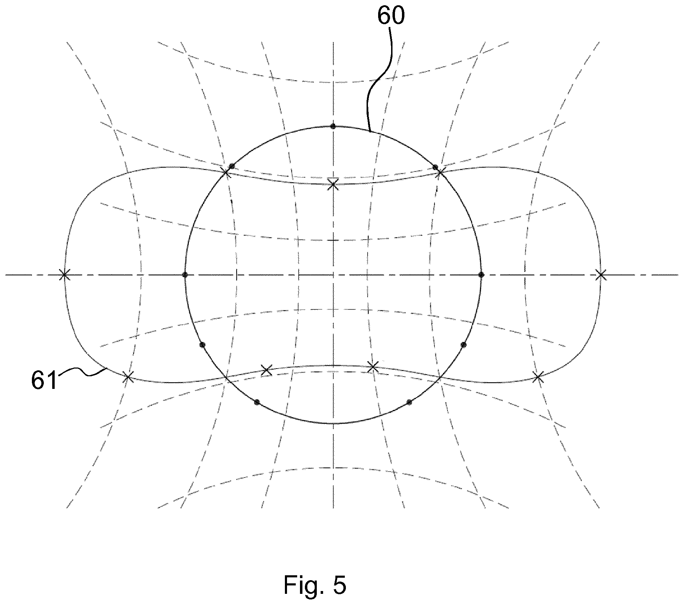

The case of shape changing/bending of the examined organ with shape change/bending considered not only on the perimeter but also regarding the inner portions of the bodymark or map 2, can be seen in Figure 5.

Figure 5 shows a graphical representation of a possible deformation algorithm, obtained with the "deformation lines" shaped on a sphere compressed along the z axis, i.e. the axis perpendicular to the drawing plane.

This is a dimensional improvement with respect to the method shown in figures 3 and 4. In figures 3 and 4 the deformation is computed along the perimeter of the breast and the inside of the breast is not taken into consideration. In figure 5, a deformation of the plane is considered, so that the scanning of the interior part of the geometry is updated in real time, considering the probe's spatial position with the x-y translation due to the pressure curve.

The first line 60 represents normal breast in supine position and the second line 61 represents the breast when it is compressed under mammography. Is it possible to consider only a part of the diagram.

If the probe is positioned on a point of line 60, by applying a transformation corresponding to a curve of pressure correction, the indication of the probe will be given on the corresponding point on the second line 61.

A family of curves can be defined in experimental way and the selection can be carried out manually by the user or simply by identifying one or two or three markers, and the selection will be done automatically selecting the best interpolation curve between the points in the two directions x-y

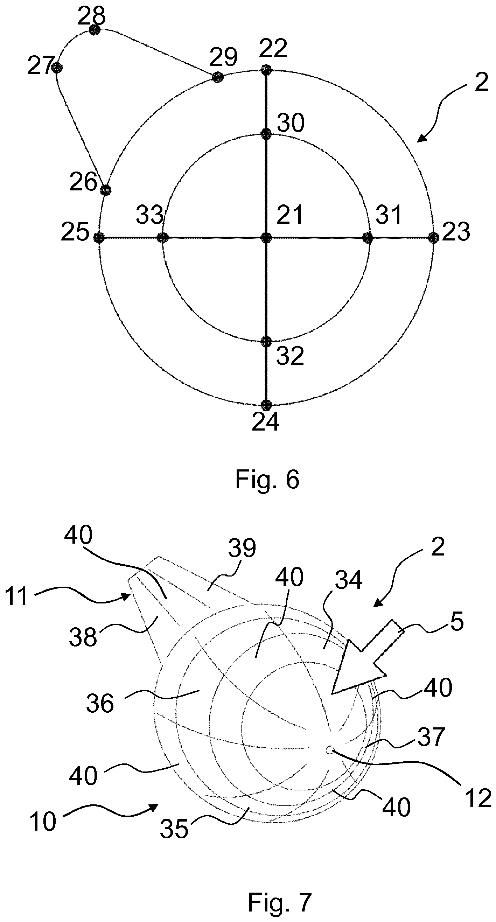

Figure 6 shows a first method of reconstructing a three-dimensional map, in which, instead of marking the nipple 12 and the above mentioned four perimeter points (inner, lower, lateral, underarm points), the operator marks 13 points, from 21 to 33 as shown in the figure.

The operator shall mark the points in the correct numbered sequence, to allow the system to automatically reconstruct a segmented three-dimensional qualitative shape of the breast 10 being examined, corresponding to that of Figure 7.

This three-dimensional view allows the operator to view the relative position of the ultrasound probe, as indicated by the arrow icon 5 or any other symbol on the reconstructed three-dimensional map, possibly in combination with the additional images and/or with a two-dimensional map.

The map of Figure 6, which has the main purposes of indicating the sequence of marking steps, may be in turn a two-dimensional map 2, on which the position of the probe at the time of ultrasound image acquisition may be marked.

Furthermore, a plurality of ultrasound images of the same anatomic region may be acquired by the probe 4, in different positions.

Each image is stored with the information about the position of the probe at the time of acquisition.

Therefore a first ultrasound image 1 is displayed with the map 2, on which the position of the probe 4 is marked by the icon 5. The map further comprises a plurality of highlighted points, corresponding to the positions of the probe 4 for each additionally acquired image.

The operator may select the highlighted points to display the acquired images corresponding to that position of the probe.

When a new image is displayed, the icon 5 is displaced to the highlighted point that has been selected.

In a further exemplary embodiment, as shown in Figure 7, the three-dimensional map that represents the volume of the breast 10 being examined and of the underarm region 11 is reconstructed by acquiring at least one three-dimensional ultrasound image of the breast and by only retaining the surface information.

Preferably, two or more three-dimensional ultrasound images are acquired and merged.

The probe for acquisition of three-dimensional ultrasound images is preferably designed to communicate with the tracking system and is manually displaced for scanning an acquisition volume.

The merging process provides wider panoramic images, that can cover a larger volume.

The system does not entirely reconstruct the scanned volumes, i.e. including internal tissue information, but only displays a reconstructed surface, consisting of the different surfaces of the real breast of the patient as scanned by the probe.

The operator moves the probe on the breast under examination to allow the system to reconstruct the surfaces of the real breast as a segmented three-dimensional model, without acquiring ultrasound volumes, but only surfaces, as sets of points.

The ultrasound probe, in communication with the tracking system, transfers data to the latter concerning its position and tilt relative to the reference system of the tracking system during surface acquisition, thereby allowing three-dimensional map reconstruction.

Acquisitions may be made in any manner, but preferably follow a predetermined time sequence.

In the embodiment of Figure 7, six successive acquisitions are made on six distinct surfaces, i.e. upper 34, lower 35, right 36, left 37, first axillary 38 and second axillary 39 surfaces.

These acquisitions were found to adequately allow reconstruction of the entire three-dimensional surface of the breast, a corresponding interpolation being provided for easy reconstruction of the surfaces of the rest of the breast.

In a preferred embodiment the logical sequence of actions in order to reconstruct the entire scanned organ surface comprise scanning consequently surfaces 34 to 39 and interpolating the geometrical information obtained for the remaining surfaces 40.

This procedure allows the remaining surfaces 40 to be reconstructed by interpolation, so that the whole surface of the breast and the axilla region is reconstructed.

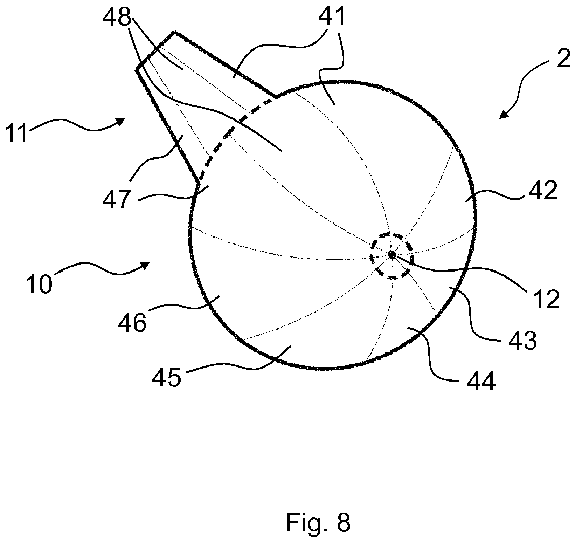

As an alternative a graphical scanning protocol can be used, as shown in figure 8, which represents a visual control/reminder for the operator in order to check/remember which parts of a certain organ to be scanned and represented on a map, have to be scanned or completed scanning.

The protocol comprise a sequence of scanned areas 41 to 48 and their related visualization or mark on the map 2, which map is previously acquired with the surface rendering technique, i.e. the three-dimensional panoramic view reconstructing only the surface of the organ or structure scanned.

This marking of the areas where the examination is performed, enables the operator to recognize which are the areas or portions of the breast 10 which still have to be examined, in order not to forget any part of the organ to be scanned.

This function can be particularly valuable for breast scanning due to the fact that the breast have a few of reference points/signs in order to remember/recognize if it was completely or partially scanned.

## Claims

1. A method of ultrasound image acquisition, generation and display, comprising the steps of:
a) acquiring ultrasound image data corresponding to an anatomic region being examined using an ultrasound probe (4);
b) generating at least one ultrasound image (1) based on said image data;
c) displaying said image (1) on a display; wherein the method further comprises the steps of:
d) generating a map (2) of the outer surface of the anatomic region;
e) detecting the position of said probe (4) during image data acquisition;
f) identifying on the map (2) the point (3) corresponding to the detected position of said probe (4) ;
g) displaying the map (2) in which said point (3) is marked, at the same time as the ultrasound image (1) is displayed, and **characterised in that** one or more additional images (6, 7) are displayed at the same time as the ultrasound image (1) and the map (2), the detected position of the above mentioned probe (4) being marked on said one or more additional images (6, 7).

2. A method as claimed in claim 1, wherein the step of generating the map (2) comprises detecting the spatial relationship among points of interest on the outer surface of said anatomic region and reproducing such spatial relationship among the points on the map (2) corresponding to said points of interest.

3. A method as claimed in claim 1, wherein the map (2) is a two-dimensional map.

4. A method as claimed in claim 3, wherein the additional images (6, 7) are acquired along two planes more or less perpendicular to the plane represented by the map (2), and perpendicular to each other.

5. A method as claimed in claim 1, wherein the map (2) is a three-dimensional map.

6. A method as claimed in claim 5, wherein the map (2) is generated by acquiring at least one three-dimensional ultrasound image of the anatomic region, and only retaining the surface information.

7. A method as claimed in claim 6, wherein two or more three-dimensional ultrasound images are acquired and merged.

8. An apparatus for ultrasound image acquisition, generation and display, comprising an ultrasound probe (4), a unit for transmitting exciting pulses and receiving image data, which is connected to said probe (4), a unit for generating ultrasound images from said image data, a display for displaying said images, a system for tracking said probe, wherein it comprises a processing unit, which is configured to generate a map (2) of the anatomic region, to locate the point (3) on the map (2) corresponding to the position of said probe (4) during image data acquisition, as detected by said tracking system and to display the ultrasound image (1) and the map (2) with said point (3) marked thereon on the display, and **characterised in that** the processing unit is further configured to display one or more additional images (6, 7) at the same time as the ultrasound image (1) and the map (2), the detected position of the above mentioned probe being marked on said one or more additional images (6, 7).

9. An apparatus as claimed in claim 8, **characterized in that** said processing unit is configured to operate as claimed in one or more of claims 2 to 7.

## Patentansprüche

1. Verfahren zur Ultraschallbilderfassung, -erzeugung und - anzeige, mit den Schritten:
a) Erfassen von Ultraschallbilddaten, die einem anatomischen Bereich entsprechen, der unter Verwendung einer Ultraschallsonde (4) untersucht wird;
b) Erzeugen mindestens eines Ultraschallbildes (1) basierend auf den Bilddaten;
c) Anzeigen des Bildes (1) auf einer Anzeige;
wobei das Verfahren ferner die Schritte umfasst:
d) Erzeugen einer Karte (2) der Außenfläche des anatomischen Bereichs;
e) Ermitteln der Position der Sonde (4) während der Bilddatenerfassung;
f) Identifizieren des Punktes (3), welcher der erfassten Position der Sonde (4) entspricht, auf der Karte (2);
g) Anzeigen der Karte (2), in der dieser Punkt (3) markiert ist, und zugleich Anzeigen des Ultraschallbildes (1), und **dadurch gekennzeichnet, dass** ein oder mehrere Zusatzbilder (6, 7) gleichzeitig mit dem Ultraschallbild (1) und der Karte (2) angezeigt werden, wobei die erfasste Position der genannten Sonde (4) auf dem einen oder den mehreren weiteren Bildern (6, 7) markiert wird.

2. Verfahren nach Anspruch 1, wobei der Schritt des Erzeugens der Karte (2) das Erfassen der räumlichen Beziehung zwischen Punkten von Interesse auf der äußeren Oberfläche des anatomischen Bereichs und das Wiedergeben einer solchen räumlichen Beziehung zwischen den Punkten auf der Karte (2), die den interessierenden Punkten entsprechen, umfasst.

3. Verfahren nach Anspruch 1, wobei die Karte (2) eine zweidimensionale Karte ist.

4. Verfahren nach Anspruch 3, wobei die Zusatzbilder (6, 7) entlang zweier Ebenen mehr oder weniger senkrecht zu der durch die Karte (2) repräsentierten Ebene und senkrecht zueinander erfasst werden.

5. Verfahren nach Anspruch 1, wobei die Karte (2) eine dreidimensionale Karte ist.

6. Verfahren nach Anspruch 5, wobei die Karte (2) durch Erfassen mindestens eines dreidimensionalen Ultraschallbildes des anatomischen Bereichs erzeugt wird und nur die Oberflächeninformationen behält.

7. Verfahren nach Anspruch 6, wobei zwei oder mehr dreidimensionale Ultraschallbilder erfasst und zusammengeschmolzen werden.

8. Vorrichtung zur Ultraschallbilderfassung, -erzeugung und -anzeige, umfassend eine Ultraschallsonde (4), eine mit der Sonde (4) verbundene Einheit zur Übertragung von Anregungspulsen und Empfangen von Bilddaten, eine Einheit zum Erzeugen von Ultraschallbildern aus den Bilddaten, eine Anzeige zum Anzeigen der Bilder, ein System zum Verfolgen der Sonde, wobei eine Verarbeitungseinheit vorgesehen ist, die dazu eingerichtet ist, eine Karte (2) der anatomischen Region zu erzeugen, um den Punkt (3) auf der Karte (2) zu lokalisieren, der der Position der Sonde (4) während der Bilddatenerfassung entspricht, wie sie durch das Verfolgungssystem erfasst wird, und um das Ultraschallbild (1) und die Karte (2) mit dem darauf markierten Punkt (3) auf der Anzeige anzuzeigen, und **dadurch gekennzeichnet, dass** die Verarbeitungseinheit ferner eingerichtet ist, ein oder mehrere zusätzliche Bilder (6, 7) gleichzeitig mit dem Ultraschallbild (1) und der Karte (2) anzuzeigen, wobei die erfasste Position der oben erwähnten Sonde auf dem einen oder den mehreren zusätzlichen Bildern (6, 7) markiert ist.

9. Vorrichtung nach Anspruch 8, **dadurch gekennzeichnet, dass** die Verarbeitungseinheit dazu eingerichtet ist, nach einem oder mehreren der Ansprüche 2 bis 7 zu arbeiten.

## Revendications

1. Une méthode pour l'acquisition, la génération et l'affichage d'images par ultrasons, comprenant les étapes de:
a) acquérir des données d'image par ultrasons correspondant à une région anatomique examinée en utilisant une sonde à ultrasons (4);
b) générer au moins une image par ultrasons (1) sur la base desdites données d'image;
c) afficher ladite image (1) sur un écran;
dans laquelle la méthode comprend en outre les étapes de:
d) générer une carte (2) de la surface externe de la région anatomique;
e) détecter la position de ladite sonde (4) lors de l'acquisition des données d'image;
f) identifier sur la carte (2) le point (3) correspondant à la position détectée de ladite sonde (4)
g) afficher la carte (2) dans lequel ledit point (3) est indiqué, au même moment que l'image par ultrasons (1) est affichée, et **caractérisée en ce qu'**une ou plusieurs images supplémentaires (6, 7) soient affichées en même temps que l'image par ultrasons (1) et la carte (2), la position détectée de la sonde (4) mentionnée ci-dessus étant indiquée sur ladite une ou plusieurs images supplémentaires (6, 7).

2. Une méthode selon la revendication 1, dans laquelle l'étape de générer la carte (2) comprend la détection de la relation spatiale entre les points d'intérêt sur la surface externe de ladite région anatomique et la reproduction de ladite relation spatiale entre les points sur la carte (2) correspondantes auxdits points d'intérêt.

3. Une méthode selon la revendication 1, dans laquelle la carte (2) est une carte bidimensionnelle.

4. Une méthode selon la revendication 3, dans laquelle les images supplémentaires (6, 7) sont acquises selon deux plans plus ou moins perpendiculaires au plan représenté par la carte (2), et perpendiculaires l'un par rapport à l'autre.

5. Une méthode selon la revendication 1, dans laquelle la carte (2) est une carte tridimensionnelle.

6. Une méthode selon la revendication 5, dans laquelle la carte (2) est générée en acquérant au moins une image par ultrasons tridimensionnelle de la région anatomique, et ne conservant que les informations de surface.

7. Une méthode selon la revendication 6, dans laquelle deux ou plusieurs images par ultrasons tridimensionnelles sont acquises et combinées.

8. Un appareil pour l'acquisition, la génération et l'affichage d'images par ultrasons, comprenant une sonde à ultrasons (4), une unité pour transmettre des impulsions d'excitation et pour recevoir des données d'image qui est connectée à ladite sonde (4), une unité pour générer des images par ultrasons à partir desdites données d'image, un écran pour afficher lesdites images, un système pour poursuivre ladite sonde, dans lequel il comprend une unité de traitement, configurée pour générer une carte (2) de la région anatomique, afin de localiser le point (3) sur la carte (2) correspondant à la position de ladite sonde (4) lors de l'acquisition des données d'image, telle que détecté par ledit système de poursuite et pour afficher l'image par ultrasons (1) et la carte (2) avec ledit point (3) indiqué sur l'affichage, et **caractérisé en ce que** l'unité de traitement est en outre configurée pour afficher une ou plusieurs images supplémentaires (6, 7) en même temps que l'image par ultrasons (1) et la carte (2), la position détectée de la sonde mentionnée ci-dessus étant indiquée sur ladite une ou lesdites plusieurs images supplémentaires (6, 7).

9. Un appareil selon la revendication 8,
**caractérisé en ce que** ladite unité de traitement est configurée pour fonctionner selon l'une ou plusieurs des revendications 2 à 7.
